# EUROPEAN PATENT APPLICATION

(11) **EP 2 659 782 A1**
(43) Date of publication of application: **06.11.2013**
(21) Application number: 12166852.9
(22) Date of filing: 04.05.2012
(51) Int. Cl.: A23F 5/02, A61K 31/216, A61K 36/185

(54) **Methods of improving mental or health conditions**

(71) Applicant: Nestec S.A., 1800 Vevey (CH)
(72) Inventor: Silber, Yvonne Beata, 1072 Forel (CH); Schmitt, Jeroen Antonius Johannes, 1510 Moudon (CH)
(74) Representative: Lomholt, Stig Bredsted

(57) **Abstract**

Methods of improving mental or health conditions of an individual are provided. In a general embodiment, the method provides administering to an individual in need of an improved mental or health condition a decaffeinated coffee product. The methods can be, for example, for improved alertness in the individual, improved attention, improved reaction time, reduction in the individual's jitteriness, tension, tiredness, mental fatigue and/or headache.

## Description

### BACKGROUND

The present disclosure generally relates to methods for improving mental or physical health conditions of an individual. More specifically, the present disclosure relates to methods of improving attention, alertness, and/or reaction times, and methods of reducing jitteriness, tension, tiredness, mental fatigue, and/or headaches of an individual.

Coffee is a complex mixture of many hundreds of compounds, which, in combination, form a unique and pleasing aroma and taste desired by many consumers. Furthermore, coffee is consumed not only for its desirable flavor but often for other reasons, such as to increase short term mental alertness. The positive health impact of coffee has been studied over many decades and, for a long time, it has been known that certain coffee compounds are capable of providing benefits to the consumer, especially increased mental alertness through the ingestion of caffeine. However, it is less well known to consumers that certain coffee compounds are excellent anti-oxidants and that, weight for weight, coffee can potentially provide significantly more antioxidants to the consumer than, for example, a well known source of antioxidants such as green tea.

A very important source of antioxidants that has been identified in coffee is the class known as chlorogenic acids. Chlorogenic acids ("CGA") in coffee are mainly mono- and di-esters of quinic acid and phenolic groups (e.g., caffeic, ferulic, coumaric, methoxycinnamic) attached to different positions. In vitro studies have confirmed the antioxidant properties of CGA, which prevents oxidation by chelating metals and scavenging reactive oxygen species. In addition to its antioxidant effects, rodent models have also shown CGA compounds to have anxiolytic, anti-inflammatory, analgesic and antipyretic effects, weak caffeine-like psychostimulant actions on spontaneous locomotor activity. A distinct neuroprotective action of CGA compounds is also evidenced by its ability to protect neuronal cells from beta-amyloid-induced toxicity and oxidative stress.

From the health and nutritional perspective, it is desirable that consumers should be able to benefit from the positive health aspects of coffee identified above and so it would be highly advantageous to utilize the beneficial components of coffee such as chlorogenic acids. However, few studies have been performed to show the impact these beneficial components on a variety of mental and physical health conditions of an individual.

### SUMMARY

The present disclosure provides methods for improving the mental or health conditions in an individual. In an embodiment, the present disclosure provides a method of improving alertness, improving attention, reducing tension, and/or reducing mental fatigue in an individual for at least 1 hour, the method comprising: administering to an individual in need of improved alertness, improved attention, reduced tension, and/or reduced mental fatigue a decaffeinated coffee product to improve alertness, improve attention, reduce tension, and/or reduce mental fatigue in the individual. The decaffeinated coffee product can have a similar effect in the individual as an approximately equivalent amount of a caffeinated coffee product.

In another embodiment, the present disclosure provides a method of improving reaction time, reducing tiredness and/or reducing jitteriness in an individual, the method comprising: administering to an individual in need of improved reaction time, reduced tiredness and/or reduced jitteriness a decaffeinated coffee product to improve reaction time, reduce tiredness and/or reduce jitteriness in the individual. The decaffeinated coffee product can have a similar effect in the individual as an approximately equivalent amount of a caffeinated coffee product.

In an alternative embodiment, the present disclosure provides a method of reducing headache for at least 1 hour in an individual, the method comprising: administering to an individual in need of reduced headache a decaffeinated coffee product to reduce headache in the individual. The decaffeinated coffee product can have a similar effect in the individual as an approximately equivalent amount of a caffeinated coffee product.

In any of the methods described herein, the decaffeinated coffee product may include a mixture of unroasted coffee and roasted coffee. In any of the methods described herein, the unroasted coffee may be in the form of an extract of an unroasted coffee. For example, the extract can be derived from (i) a first portion including unroasted ground and/or unground coffee, in an amount of from 1 to 90% by weight based on the total weight of the coffee product, and (ii) a second portion including ground coffee that has been roasted to a higher degree of roast than the first portion, in an amount of from 99 to 10% by weight based on the total weight of the coffee product, wherein the content of the chlorogenic acids is at least 4 g per 100 g of the coffee product.

In yet another embodiment, the present disclosure provides a method of improving alertness in an individual for at least 1 hour, the method comprising: administering to an individual in need of improved alertness a non-coffee product comprising a component selected from the group consisting of an unroasted coffee extract, chlorogenic acids and combinations thereof to improve alertness in the individual.

In still another embodiment, the present disclosure provides a method of reducing jitteriness in an individual, the method comprising: administering to an individual in need of reduced jitteriness a non-coffee product comprising a component selected from the group consisting of an unroasted coffee extract, chlorogenic acids and combinations thereof to reduce jitteriness of the individual.

In another embodiment, the present disclosure provides a method of reducing headache for at least 1 hour in an individual, the method comprising: administering to an individual in need of reduced headache a non-coffee product comprising a component selected from the group consisting of an unroasted coffee extract, chlorogenic acids and combinations thereof to reduce headache of the individual.

In any of the methods herein, the chlorogenic acids can be derived from a food source (including coffee). The non-coffee product can be a food product, a beverage, a food supplement, a pet care product, a pharmaceutical product or a combination thereof.

In an embodiment, the present disclosure provides the use of a decaffeinated coffee product to improve alertness, improve attention, reduce tension, and/or reduce mental fatigue in an individual for at least 1 hour. In another embodiment, the present disclosure provides the use of a decaffeinated coffee product to reduce tiredness, improve reaction time, and/or reduce jitteriness in an individual. In yet another embodiment, the present disclosure provides the use of chlorogenic acids to reduce jitteriness in an individual. In yet another embodiment, the present disclosure provides the use of chlorogenic acids to reduce headache in an individual for at least 1 hour. In yet another embodiment, the present disclosure provides the use of chlorogenic acids to reduce tiredness in an individual for at least 1 hour.

An advantage of the present disclosure is to provide a method of improving a mental condition of an individual using a decaffeinated coffee product.

Another advantage of the present disclosure is to provide a method of improving a health condition of an individual using a decaffeinated coffee product.

Still another advantage of the present disclosure is to provide a method of improving a mental or health condition of an individual using chlorogenic acids.

Yet another advantage of the present disclosure is to provide a method of improving the alertness, and/or reducing jitteriness, tension, tiredness, headache and/or mental fatigue of an individual using an extract from a coffee product.

Additional features and advantages are described herein, and will be apparent from, the following Detailed Description.

### DETAILED DESCRIPTION

The present disclosure relates generally to methods for improving the mental and/or health conditions of an individual. In a general embodiment, the method provides administering to an individual in need of an improved mental or health condition a decaffeinated coffee product. The decaffeinated coffee product can include portions of an unroasted and a roasted coffee in any suitable form.

Without being bound by theory, it has been surprisingly found that providing individuals with components of a decaffeinated coffee product can improve various mental and health conditions in an individual. The mental or health condition can be, for example, improved alertness in the individual, improved attention, improved reaction time, reduced jitteriness, reduced tension, reduced tiredness, reduced headache and/or reduced mental fatigue. These improved mental or health conditions can include those not normally attributable to caffeine typically found in coffee products.

As used herein, the term "alertness" refers to the state of paying close and continuous attention, being watchful and prompt, or being quick to perceive and/or act. It is being vigilantly attentive, mentally responsive and perceptive, and quick.

As used herein, the term "reaction time" refers to the elapsed time between the onset of a stimulus and the detection of a response to the stimulus.

As used herein, the term "jitteriness" refers to feeling nervous or uneasy. Can be marked by jittering movements.

As used herein, the term "tension" refers to mental, emotional, or nervous strain.

As used herein, the term "tiredness" refers to exhausted of strength and/or energy; fatigued or sleepy.

As used herein, the term "mental fatigue" refers to a state of awareness describing a range of afflictions, associated with mental weakness. Such mental fatigue can manifest itself either as somnolence (decreased wakefulness) or as a general decrease of attention (not necessarily sleepiness). It may also be described as a decreased level of consciousness. Mental fatigue can be caused by continual mental effort and attention on a particular task, as well as high levels of stress or emotion. Basically, any mental process that goes into overload can result in mental fatigue.

As used herein, the term "headache" refers to a persistent or lasting pain or discomfort in the head region.

As used herein the term "attention" refers to the ability to direct and focus cognitive activity on specific stimuli. It is the ability or power to concentrate mentally.

As used herein, the term "decaffeinated coffee product" refers to a coffee product that has some, most or all of the caffeine removed (e.g., via a decaffeination process). For example, the coffee product can have a reduced amount of caffeine (e.g., 95% removal, 97% removal, 99% removal) compared to the amount normally associated with that coffee product.

As used herein a "non-coffee product" is a product which is not based on coffee or coffee extract as the main ingredient, but which may contain coffee components or extracts, e.g. chlorogenic acids, as a minor compound, or may contain a purified fraction of coffee, e.g. purified chlorogenic acids.

Chlorogenic acids have the general formula:

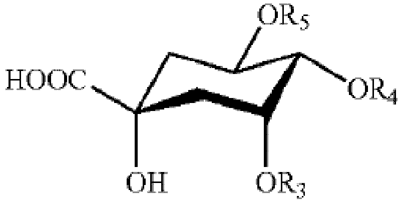

The most prevalent chlorogenic acids are given in the following table.

| Name | Structure Chlorogenic acids | R3 | R4 | R5 |
|---|---|---|---|---|
| 5-O-caffeoyl-D-quinic acid (5CQA) | C | H | H | caffeoyl |
| 4-O-caffeoyl-D-quinic acid (4CQA) | C | H | caffeoyl | H |
| 3-O-caffeoyl-D-quinic acid (3CQA) | C | caffeoyl | H | H |

Other isomers exist which also fall within the definition of the structure given above. Therefore, in the context of the present disclosure, the phrase "chlorogenic acids" denotes all chlorogenic acid isomers, which are defined by the aforementioned structure.

In an embodiment, the present disclosure provides a method of improving alertness, improving attention, reducing tension, and/or reducing mental fatigue in an individual for at least 1 hour, preferably for at least 2 hours. The method comprises administering to an individual in need of improved alertness, improved attention, reduced tension, and/or reduced mental fatigue a decaffeinated coffee product to improve alertness, improve reaction attention, reduce tension, and/or reduce mental fatigue in the individual. The decaffeinated coffee product can be partially or fully decaffeinated. The decaffeinated coffee product can have a similar effect in the individual as an approximately equivalent amount of a caffeinated coffee product.

In another embodiment, the present disclosure provides a method of improving reaction time, reducing tiredness and/or reducing jitteriness in an individual, the method comprising: administering to an individual in need of improved reaction time, reduced tiredness and/or reduced jitteriness a decaffeinated coffee product to reduce tiredness, improve reaction time and/or reduce jitteriness in the individual, preferably for at least 1 hour, more preferably for at least 2 hours. The decaffeinated coffee product can be partially or fully decaffeinated. The decaffeinated coffee product can have a similar effect in the individual as an approximately equivalent amount of a caffeinated coffee product.

In an alternative embodiment, the present disclosure provides a method of reducing headache for at least 1 hour, preferably for at least 2 hours, in an individual, the method comprising: administering to an individual in need of reduced headache a decaffeinated coffee product to reduce headache in the individual. The decaffeinated coffee product can be partially or fully decaffeinated. The decaffeinated coffee product can have a similar effect in the individual as an approximately equivalent amount of a caffeinated coffee product.

The following discussion of the decaffeinated coffee products and extracts applies to any embodiments of the present disclosure. In an embodiment, the decaffeinated coffee product can include unroasted coffee, or roasted coffee, or a mixture of unroasted coffee and roasted coffee. In another embodiment, the decaffeinated coffee product can include a high content of chlorogenic acids or an amount of chlorogenic acids beyond the amount naturally associated with a roasted coffee product. In an alternative embodiment, the decaffeinated coffee product can include an amount of chlorogenic acids ranging from about 100 mg to about 10,000 mg per serving, including for example, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1,000 mg, 2,000 mg, 3,000 mg, 4,000 mg, 5,000 mg, 6,000 mg, 7,000 mg, 8,000 mg, 9,000 mg and the like and any amount therebetween. Preferably, the decaffeinated coffee product can include an amount of chlorogenic acids ranging from about 100 mg to about 5000 mg, such as from about 150 mg to about 1000 mg, per serving.

In another embodiment, the decaffeinated coffee is in the form of an extract. The extract can be derived from roasted coffee, unroasted coffee, (e.g., green coffee beans), or a mixture of unroasted coffee and roasted coffee (e.g., beans). Details of such an extract can be found, for example, in U.S. Patent Serial No. 11/911,046, which is incorporated herein by reference. In an embodiment, the extract can be derived from (i) a first portion including unroasted ground and/or unground coffee, in an amount of from 1 to 90% by weight based on the total weight of the coffee product, preferably in an amount of from 20% to 50%; and (ii) a second portion including ground coffee that has been roasted to a higher degree of roast than the first portion, in an amount of from 99 to 10% by weight based on the total weight of the coffee product, preferably from 80% to 50%. The content of the chlorogenic acids in the decaffeinated coffee product is preferably at least 4 g per 100 g of dry solids of the coffee product.

In any embodiments of the present disclosure, the extract can be derived from a mixture of green (e.g., unroasted) and roasted coffee beans in which the weight ratio of the green portion and roasted portion in the coffee product is from 1:99 to 90:10, alternatively from 20:80 to 50:50, alternatively from 30:70 to 45:55.

In an embodiment, the green coffee portion is substantially or wholly derived from Robusta coffee beans. In another embodiment, at least 65%, more preferably 75%, even more preferably 85%, most preferably 95%, for example 100% by weight of the green coffee beans are Robusta. This is because it has been found that Robusta coffee beans provide a greater level of chlorogenic acid per gram of bean than Arabica beans.

The roasted coffee portion may be a blend of Arabica and Robusta, although, in a preferred embodiment, the roasted portion is substantially or wholly derived from Arabica beans. This is because Arabica provides a richer taste and aroma profile, which is associated with coffee of higher quality.

The green coffee portion and/or the roasted coffee portion is partially or fully decaffeinated. However, it is particularly preferred that the green coffee portion is from fully decaffeinated coffee. Preferably the coffee product is used to provide a soluble coffee product.

The coffee beans to be extracted may be whole or ground. In an embodiment, green coffee beans are co-extracted with roasted coffee beans, i.e., green and roasted coffee beans are extracted simultaneously in the same extraction system to yield a mixed extract. The most volatile aroma components can be stripped from the beans before extraction, for example, if the extract is to be used for the production of pure soluble coffee. Methods for stripping of volatile aroma components are well known in the art, for example, from EP 1078576, which is incorporated herein by reference.

Following stripping, the less volatile components are then subjected to an "extraction" step. Extraction is a term common in the art of processing soluble coffee and indicate a process where water and/or steam are used to extract a complex mixture of coffee components from the roasted, ground coffee bean.

Extraction of coffee beans can be done using any suitable extraction method known to the skilled artisan. For example, extraction can be performed using water and/or steam as described in EP 0916267, which is incorporated herein by reference. In an embodiment, the coffee beans may be extracted by any suitable method yielding an extract including a desired amount of chlorogenic acids.

After extraction, the product may undergo a concentration step, following which, if a stripping step has taken place, the stripped aroma can be reintroduced into the resulting extract. Finally, the aromatized extract can be dried according to any standard procedure, such as freeze-drying, spray-drying or agglomerating. This produces a solid soluble product that may suitably be in the form of a powder or granules. If the extract has been dried, it may be resuspended as necessary for administration.

The soluble coffee mixture may be produced by co-extraction of the green portion and the roasted portion. Alternatively, the soluble coffee product can be produced by separately providing a roasted coffee portion and a green coffee portion and then extracting them individually before combining the resultant extracts. This is advantageous as the extraction conditions can be adapted to suit roasted and unroasted beans respectively.

The coffee extract may undergo any suitable treatment to remove undesired components of the extract. The extract may be separated from the extracted coffee beans by any suitable method, for example, filtration or centrifugation. The separation may be performed to the extent practically and economically feasible and needed in view of the desired use of the extract. The separation may thus not be 100% complete, for example, a minor part of undissolved material from the beans may still be present with the extract after separation.

In yet another embodiment, the present disclosure provides a method of improving alertness in an individual for at least 1 hour, the method comprising: administering to an individual in need of improved alertness a non-coffee product comprising a component selected from the group consisting of a decaffeinated coffee extract, chlorogenic acids and combinations thereof to improve attention in the individual.

In still another embodiment, the present disclosure provides a method of reducing jitteriness in an individual, the method comprising: administering to an individual in need of reduced jitteriness a non-coffee product comprising a component selected from the group consisting of a decaffeinated coffee extract, chlorogenic acids and combinations thereof to reduce jitteriness of the individual.

In still another embodiment, the present disclosure provides a method of reducing headache for at least 1 hour in an individual, the method comprising: administering to an individual in need of reduced headache a non-coffee product comprising a component selected from the group consisting of a decaffeinated coffee extract, chlorogenic acids and combinations thereof to improve the relaxation of the individual.

In any of the methods described herein, the chlorogenic acids can be derived, for example, from any suitable food source including coffee, tea, blackberry, potato or tomato. The chlorogenic acids can be derived, for example, from any suitable non-food source such as chemical synthesis. In an embodiment, the amount of chlorogenic acids added to the non-coffee product ranges from about 100 mg to about 10,000 mg per serving, including for example, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1,000 mg, 2,000 mg, 3,000 mg, 4,000 mg, 5,000 mg, 6,000 mg, 7,000 mg, 8,000 mg, 9,000 mg and the like and any amount therebetween. Preferably, the amount of chlorogenic acids added to the non-coffee product ranges from about 100 mg to about 5000 mg, such as from about 150 mg to about 1000 mg, per serving. The non-coffee product can be a food product, a beverage, a food supplement, a pet care product, a pharmaceutical product or a combination thereof.

In an alternative embodiment, the present disclosure provides a method of making a consumable product for improving an individual's mental capabilities. The method comprises providing a decaffeinated coffee extract for improving the individual's mental capabilities, and adding the decaffeinated coffee extract to a consumable product. The decaffeinated coffee extract may include an effective amount of chlorogenic acids for improving the individual's mental capabilities. The consumable product can be a food product, a beverage, a food supplement, a pet care product, a pharmaceutical product or a combination thereof. The mental capabilities can be alertness, mood, attention or a combination thereof.

In any embodiments of the present disclosure, the decaffeinated coffee extract can be used separately from the extracted coffee beans. The undissolved material from the beans can be substantially removed by separation as described herein and is not used in the production of the food or beverage product.

The decaffeinated coffee extract and/or chlorogenic acids can be added as an ingredient in any suitable form (e.g., liquid, powder, granules, etc.) to any suitable consumable product. In an embodiment, the consumable product may be any food or beverage product known in the art. For example, the food or beverage products can be a coffee beverage, pure soluble coffee, a soft drink, a dietary supplement, a dairy product, a cereal product, a fruit or vegetable juice product, or a confectionary product, such as a chocolate product, for example a chocolate drink. A soluble coffee product may be produced by concentrating and drying the decaffeinated coffee extract. A soluble coffee product produced from the decaffeinated coffee extract may be sold as such, or may, for example, be mixed with a creamer and/or sweetener and sold to prepare a coffee beverage including creamer and/or sweetener, for example, cappuccino or cafe latte.

In an embodiment, the present disclosure provides the use of a decaffeinated coffee extract to improve alertness, improve attention, reduce tension, and/or reduce mental fatigue in an individual for at least 1 hour, preferably for at least 2 hours. The decaffeinated coffee extract may comprise an extract of unroasted coffee. In another embodiment, the present disclosure provides the use of a decaffeinated coffee extract to reduce tiredness, improve reaction time and/or reduce jitteriness in an individual, preferably for at least 1 hour, more preferably for at least 2 hours. The decaffeinated coffee extract may be decaffeinated.

In still another embodiment, the present disclosure provides the use of chlorogenic acids to reduce jitteriness in an individual, preferably for at least 1 hour, more preferably for at least 2 hours. In yet another embodiment, the present disclosure provides the use of chlorogenic acids to reduce headache of an individual for at least 1 hour, preferably for at least 2 hours. In yet another embodiment, the present disclosure provides the use of chlorogenic acids to reduce tiredness in an individual for at least 1 hour, preferably for at least 2 hours. The uses of chlorogenic acids disclosed herein may be performed e.g. by administrating chlorogenic acids to an individual for oral consumption, e.g. in the form of a coffee product, a decaffeinated coffee product as disclosed herein, or a non-coffee product as disclosed herein comprising chlorogenic acids. The use may be performed by administrating from about 100 mg to about 10,000 mg of chlorogenic acids to an individual for oral consumption, including for example, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1,000 mg, 2,000 mg, 3,000 mg, 4,000 mg, 5,000 mg, 6,000 mg, 7,000 mg, 8,000 mg, 9,000 mg and the like and any amount therebetween. Preferably, the amount of chlorogenic acids administered to an individual for oral consumption ranges from about 100 mg to about 5000 mg, such as from about 150 mg to about 1000 mg.

In another embodiment, the present disclosure provides a method of increasing sports performance in an individual for at least 1 hour, preferably for at least 2 hours. The method comprises administering to an individual in need thereof a decaffeinated coffee product comprising an unroasted coffee to increase the sports performance in the individual. In an alternative embodiment, the present disclosure provides a method of increasing sports performance in an individual for at least 1 hour, preferably for at least 2 hours. The method comprises administering to an individual in need of thereof a non-coffee product comprising a component selected from the group consisting of a decaffeinated coffee extract, chlorogenic acids and combinations thereof to increase the sports performance in the individual.

Caffeine is known to improve sports performance by improving attention, increasing alertness, and reducing mental fatigue (thus the improvement comes directly from improving cognitive function). Accordingly, improvements in cognitive processes as discussed herein are the same attributes that have been known to improve sports performance (e.g., alertness, sustained attention, reducing mental fatigue).

### EXAMPLES

By way of example and not limitation, the following examples are illustrative of various embodiments of the present disclosure.

### EXAMPLE 1

Cognitive and mood data were collected from 66 healthy older participants (50+) who were light to moderate coffee drinkers (i.e. drinking no more than 8 cups of coffee per week). A randomised, double blind, placebo-controlled cross-over-design was employed. Each participant was tested under three acute oral coffee conditions: 1) 540 mg chlorogenic acid (equivalent to level found in 3 servings of decaffeinated Green Blend) mixed in a placebo matrix (5460mg maltodextrin and CHE mixed with coffee flavour and colour; 6g), 2) decaffeinated Green Blend coffee (6g), and 3) placebo (6g; maltodextrin and CHE mixed with coffee flavour and colour). A selected battery of cognitive tasks were performed to measure attention, emotional attention, information processing speed, reaction time performance, and mood.

### Objectives

The primary objective was to evaluate whether an acute dose of chlorogenic acid (CGA) improves cognitive function and mood in healthy older adults.

The secondary objectives were to:

1.) Evaluate whether it is the CGAs found in decaffeinated coffee that are attributed to the cognitive and mood enhancing effects;

2.) Replicate the positive acute effects of 6 grams Green Blend decaffeinated coffee on attention and mood;

### Description of tests

N-back task is a measure of sustained and selective attention and impulsivity. Single digits are presented on the screen. Participants are required to press 'YES' or 'NO' using a button box, to indicate whether the digit is the same as the n previously (e.g. 1-back the previous digit, 3-back the digit 3 previously). The task is scored for speed and accuracy. The duration of the task is approximately 4 minutes.

Jensen's Box Reaction Time task is an apparatus that distinguishes decision time and movement time from total reaction time. This apparatus has eight lights which are arranged in a semi-circular configuration. A response button is located adjacent to each light. A "home" button is situated in the centre of the panel. Subj ects are required to press the home button until they see a target light and then to release the home button as quickly as possible and to press the response button adjacent to the stimulus light. Decision time (DT) is defined as the time from stimulus onset to the release of the home button, and movement time (MT) as the time from release of the home button to the depression of the stimulus button. Choice is manipulated by varying the number of stimulus alternatives, from 0 (i.e. one light at one possible location) to multiple (i.e. the stimulus may appear in any one of the eight light positions). Participants will be given several practice trials in the eight stimulus (i.e. eight lights) condition so that they can familiarise themselves with the task. DTs of less than 150 ms are discarded as outliers, as it has been argued that physiological limits prevent shorter DTs (Jensen, 1987). DTs over 999 ms will also be discarded and replaced with an additional trial. In addition, all DTs exceeding three SDs above the subject's mean DT are also discarded (Jensen, 1987). The outcome measures are the median, mean and intra-individual variability (*i - average standard deviation) of both DT and MT for all choice, intercept of the DT function across choice and the slope of this function.

Bond and Lader Visual Analogue Scales (VAS) (Bond & Lader, 1974) is a frequently used self-evaluation mood and alertness rating scale. In total, 16 dimensions of mood are given. The participant is required to mark, on a 100 mm line to what extent the described state is appropriate to him/her at that moment in time. The Bond and Lader VAS discriminates three affective dimensions: alertness, contentment, and calmness.

Caffeine Research Visual Analogue Scales consists of seven visual analogue scales ("relaxed", "alert", "jittery", "tired", "tense", "headache", overall mood") that have previously been used in research into the effects of caffeine (Rogers et al., 2003). In addition, a single "mentally fatigued" visual analogue scale was included, as previous research has shown it to be sensitive to a caffeine-glucose drink (Kennedy & Scholey, 2004).

Cognitive performance and mood was assessed at baseline, 40 minutes and 120 minutes post treatment consumption.

### Design

This was an acute cross-over, placebo-controlled, double blind, randomized, single centre, clinical trial with three treatment conditions: chlorogenic acid, Green Blend decaffeinated coffee and placebo.

### Subjects

Sixty healthy older adults completed the study. Criteria for inclusion was: healthy older adults (male and female) aged 50+; light to moderate coffee drinkers (i.e. with caffeine), where subjects drink no more than 8 cups of coffee a week; no existing or pre-existing physical or neurological conditions, no history of psychiatric, cardiac, endocrine, gastrointestinal, or bleeding disorders, not taking any medication that could potentially affect the outcome of the study (i.e. psychoactive medication) including drugs, excessive amounts of alcohol, non-smokers, not on a potassium reduced diet, not under treatment with spironolactone-like anti-diuretics, aldosteron-receptors antagonists or angiotensin II-antagonists and no food allergies.

### Products tested

Chlorogenic acid in placebo matrix (maltodextrin and CHE mixed with coffee flavour and colour); Green Blend decaffeinated coffee; and placebo (maltodextrin and CHE mixed with coffee flavour and colour).

Amount, dosage, route of administration, duration of treatment:
- 540 mg chlorogenic acid mixed in a placebo matrix (5460mg maltodextrin and CHE mixed with coffee flavour and colour) ("CGA"). The CGA is equivalent to the level found in 3 servings of a Green Blend decaffeinated coffee
- Green Blend Decaffeinated coffee ("Green" or "Green Blend Decaf"): A decaffeinated soluble coffee product produced by co-extraction of a blend of 65% by weight of roasted coffee beans and 35% by weight of unroasted coffee beans (6g);
- Placebo (6 g maltodextrin and CHE based with coffee flavour, colour and aroma).
The composition of each coffee treatment are summarized in Table 1.

**Table 1**

| Samples | | Unit | Green Blend Decaf | Placebo | CGA |
|---|---|---|---|---|---|
| **Caffeine** | | g/100g as such | 0.08 | 0.01 | 0.16 |
| **Chlorogenic acids** | | **g/100g as such** | **8.86** | **0.00** | **9.30** |
| | 3CQA | g/100g as such | 2.01 | 0.00 | 1.52 |
| | 4CQA | g/100g as such | 2.35 | 0.00 | 2.43 |
| | 5CQA | g/100g as such | 2.58 | 0.00 | 2.56 |
| | 3,4diCQA | g/100g as such | 0.32 | 0.00 | 0.60 |
| | 3,5diCQA | g/100g as such | 0.18 | 0.00 | 0.36 |
| | 4,5diCQA | g/100g as such | 0.28 | 0.00 | 0.55 |
| | 4FQA | g/100g as such | 0.42 | 0.00 | 0.54 |
| | 5FQA | g/100g as such | 0.72 | 0.00 | 0.74 |
| | caffeic acid | g/100g as such | 0.01 | 0.00 | 0.09 |

| Per servings | | Unit | Green Blend Decaf | Placebo | CGA |
|---|---|---|---|---|---|
| **dosage** | | g/cup | 6.00 | 6.00 | 5.70 |
| **Caffeine** | | mg/cup | 5.0 | 0.7 | 8.8 |
| **Chlorogenic acids** | | mg/cup | 532 | 0 | 530 |

All treatments were administered orally as a hot coffee beverage which was drunk within 15 minutes. Participants consumed each of the coffee beverages on separate testing days. Experimental testing sessions were separated by at least a one week washout period.

### Statistical analysis

All dependent variables were tested for normality using the Box-Cox Log-likelihood plots for various values of lambda. In cases where the 95% confidence interval covered lamda = 1 the data was left as is. In cases where the confidence interval did not cover 1 the data was transformed using the appropriate box-cox power transformation. In many cases the logarithm or square root was appropriate for normalizing the data, while in isolated cases inverse or squared transformations were also used. The normality of the transformed data was subsequently confirmed using Kolmogorov-Smirnov.

All analysis of variance was conducted using SAS 9.2 with repeated measures mixed

modelling (PROC MIXED). The multivariate correlated error approach was used, whereby within-subject parameter estimates of residual covariance were estimated for each combination of time and treatment. This model was compared with a secondary model whereby each participant was treated as a random effect, and the former correlated error approach was found to result in superior fit (lower AIC value). The Residual Maximum Likelihood estimation method (REML) was used with unstructured (UN) covariance and a between-within degrees of freedom method. Fixed terms were fitted for the Treatment Effect (Placebo, Green Blend, CGA) and Time since dosing (40 mins, 120 mins). Baseline performance was fitted as a covariate and the interaction of Treatment* Visit was specified as the Repeated term.

Post-hoc differences of least squares means were compared between all levels of treatment and time. Significance values were reported using both unadjusted p-values as well as with Hommel correction of p-values for multiple comparisons. An alpha level of 0.05 was set for statistical significance of least square differences, when using the Hommel corrected p-values (highlighted in colour in tables of results). However, to allow greater exploration of the data, any least square differences associated with uncorrected p-values <.100 were taken as trend level and have also been discussed in text (highlighted in grey in tables of results).

### RESULTS

### N-Back task

| **Analysis Variable : NBack_Correct Reaction Time (ms)** | | | | | | | | | | | |
|---|---|---|---|---|---|---|---|---|---|---|---|
| | | | | | | | | **Lower 95%** | **Upper 95%** | | |
| **Treatment** | **Time** | **Obs** | **Mean** | **Median** | **N** | **Std Dev Std** | **Error CL** | **for Mean CL** | **for Mean** | **Minimum** | **Maximum** |
| ***Placebo*** | ***Pre*** | 60 | 1142 | 1019 | 60 | 409.3 | 52.84 | 1036 | 1248 | 637.1 | 2559 |
| | ***40*** | 60 | 1137 | 1030 | 60 | 445.8 | 57.55 | 1021 | 1252 | 466.3 | 2459 |
| | ***120*** | 60 | 1086 | 966.7 | 60 | 406.5 | 52.48 | 981.4 | 1191 | 601.4 | 2749 |
| ***Green*** | ***Pre*** | 60 | 1131 | 1043 | 60 | 418.1 | 53.98 | 1023 | 1239 | 598.4 | 2418 |
| | ***40*** | 60 | 1041 | 960.7 | 60 | 363.5 | 46.93 | 947.3 | 1135 | 373.4 | 2370 |
| | ***120*** | 60 | 1013 | 920.1 | 60 | 366.8 | 47.35 | 918.6 | 1108 | 294.7 | 2702 |
| ***CGA*** | ***Pre*** | 60 | 1125 | 1065 | 60 | 408.6 | 52.75 | 1020 | 1231 | 596.0 | 2431 |
| | ***40*** | 60 | 1076 | 968.9 | 60 | 452.6 | 58.43 | 958.7 | 1193 | 488.6 | 3190 |
| | ***120*** | 60 | 1048 | 952.6 | 60 | 354.6 | 45.78 | 956.4 | 1140 | 541.7 | 2304 |

Mixed ANCOVA adjusting for baseline accuracy and considering time, treatment and their interaction as independent variables were fitted. The main effect for treatment was near-significant (F-value=3.12, p-value=0.05), while the Treatment*Time interaction was non-significant (F-value=0.46, p-value=0.63).

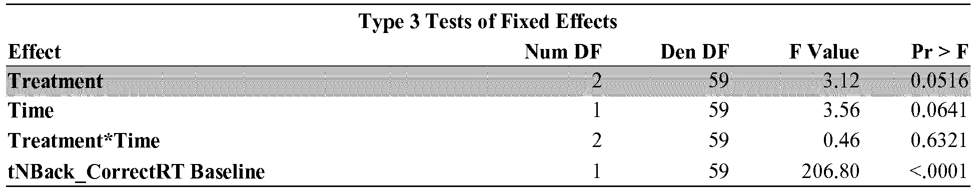

Differences of Least Square Means were calculated between the three treatments as well as the interaction of Treatment*Time. Both unadjusted and adjusted (using Hommel correction) p-values are reported.

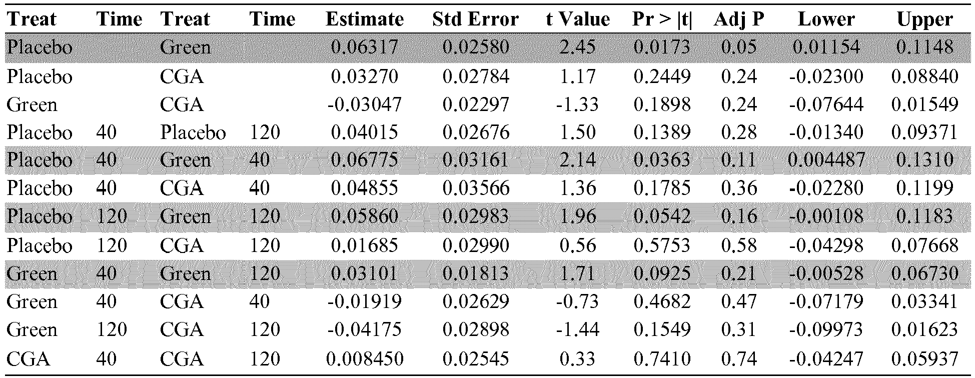

At both 40 minutes and 120 minutes post-treatment, there was a trend towards reaction time (RT) being faster in the Green Blend group in comparison to placebo (p-values = 0.11 and 0.21 respectively). There was also a trend towards a reduction in RT from 40 to 120 minutes in the Green Blend group (p-value=0.21). Overall, RT was found to be significantly faster after consuming Green Blend in comparison to Placebo (p-value=0.05).

### Jenson Box task

| **Analysis Variable(s) : Jensen Decision Time (ms)** | | | | | | | | | | | | |
|---|---|---|---|---|---|---|---|---|---|---|---|---|
| | | | | | | | | | **Lower 95%** | **Upper 95%** | | |
| | **Treat** | **Time** | **Obs** | **Mean** | **Median** | **N** | **Std Dev** | **Std Error** | **CL for Mean** | **CL for Mean** | **Minimum** | **Maximum** |
| 2-ch | ***Placebo*** | ***Pre*** | 60 | 342.9 | 338.5 | 60 | 58.00 | 7.488 | 327.9 | 357.8 | 231.0 | 504.0 |
| | | ***40*** | 60 | 354.9 | 350.0 | 60 | 54.17 | 6.993 | 340.9 | 368.9 | 218.0 | 489.0 |
| | | ***120*** | 60 | 351.3 | 344.0 | 60 | 58.14 | 7.506 | 336.3 | 366.4 | 234.0 | 554.0 |
| | ***Green*** | ***Pre*** | 60 | 354.2 | 340.5 | 60 | 64.55 | 8.334 | 337.5 | 370.8 | 249.0 | 522.0 |
| | | ***40*** | 60 | 350.7 | 340.0 | 60 | 59.66 | 7.703 | 335.3 | 366.1 | 260.0 | 475.0 |
| | | ***120*** | 60 | 356.9 | 347.5 | 60 | 58.31 | 7.527 | 341.9 | 372.0 | 250.0 | 516.0 |
| | ***CGA*** | ***Pre*** | 60 | 344.4 | 337.5 | 60 | 52.98 | 6.840 | 330.7 | 358.1 | 221.0 | 461.0 |
| | | ***40*** | 60 | 353.6 | 341.5 | 60 | 62.51 | 8.069 | 337.4 | 369.7 | 220.0 | 519.0 |
| | | ***120*** | 60 | 357.5 | 349.5 | 60 | 53.83 | 6.950 | 343.6 | 371.4 | 256.0 | 490.0 |
| 4-ch | ***Placebo*** | ***Pre*** | 60 | 363.5 | 351.5 | 60 | 60.13 | 7.762 | 348.0 | 379.0 | 225.0 | 588.0 |
| | | ***40*** | 60 | 369.4 | 358.0 | 60 | 59.37 | 7.665 | 354.1 | 384.7 | 236.0 | 548.0 |
| | | ***120*** | 60 | 372.1 | 359.0 | 60 | 64.04 | 8.268 | 355.5 | 388.6 | 260.0 | 600.0 |
| | ***Green*** | ***Pre*** | 60 | 368.9 | 351.0 | 60 | 62.26 | 8.038 | 352.8 | 385.0 | 267.0 | 532.0 |
| | | ***40*** | 60 | 365.4 | 361.5 | 60 | 56.95 | 7.353 | 350.6 | 380.1 | 253.0 | 500.0 |
| | | ***120*** | 60 | 370.4 | 364.5 | 60 | 54.98 | 7.097 | 356.2 | 384.6 | 260.0 | 506.0 |
| | ***CGA*** | ***Pre*** | 60 | 362.2 | 353.5 | 60 | 55.32 | 7.142 | 347.9 | 376.5 | 244.0 | 492.0 |
| | | ***40*** | 60 | 370.0 | 357.0 | 60 | 54.94 | 7.092 | 355.8 | 384.2 | 234.0 | 497.0 |
| | | ***120*** | 60 | 373.9 | 370.0 | 60 | 55.82 | 7.206 | 359.5 | 388.3 | 255.0 | 508.0 |

Jenson 2-choice. Mixed ANCOVA adjusting for baseline accuracy and considering time, treatment and their interaction as independent variables were fitted. The main effect for treatment was non-significant (F-value=1.87, p-value=0.16) while the Treatment*Time interaction was approaching significance (F-value=2.36, p-value=0.10).

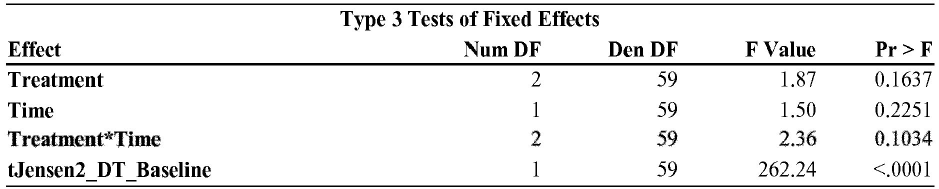

Differences of Least Square Means were calculated between the three treatments as well as the interaction of Treatment*Time. Both unadjusted and adjusted (using Hommel correction) p-values are reported.

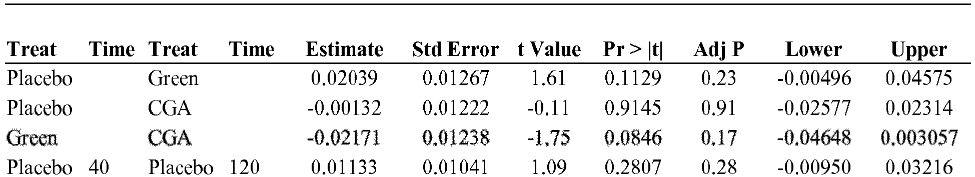

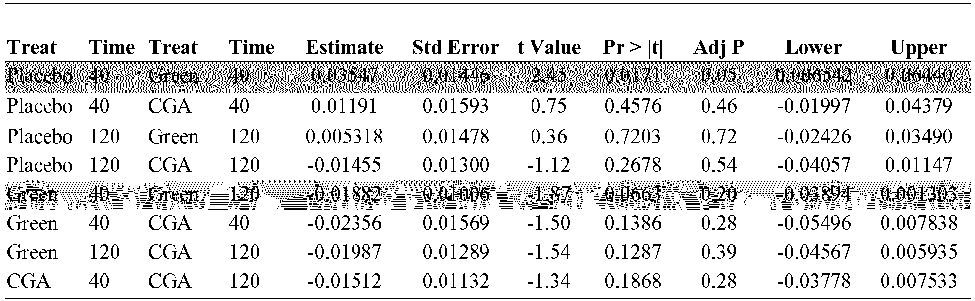

At 40 minutes post-treatment, subjects receiving Green Blend displayed significantly faster decision time (DT) in comparison to the placebo group (p-value = 0.05). There was also a trend towards DT being faster in the Green Blend group in comparison to CGA overall (p-value=0.17). From 40 to 120 minutes post treatment there was a trend towards an increase in DT in the Green Blend group (p=0.20).

Jenson 4-choice. Mixed ANCOVA adjusting for baseline accuracy and considering time, treatment and their interaction as independent variables were fitted. The main effect for treatment was approaching significance (F-value=2.58, p-value=0.08) while the Treatment*Time interaction was non-significant (F-value=0.16, p-value=0.85).

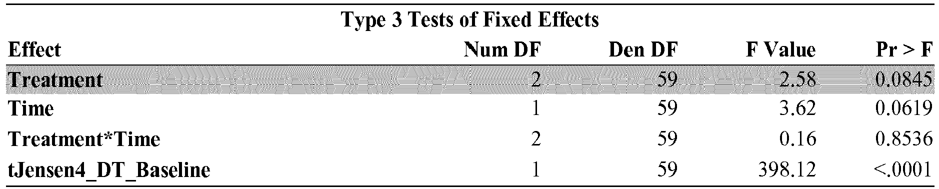

Differences of Least Square Means were calculated between the three treatments as well as the interaction of Treatment*Time. Both unadjusted and adjusted (using Hommel correction) p-values are reported.

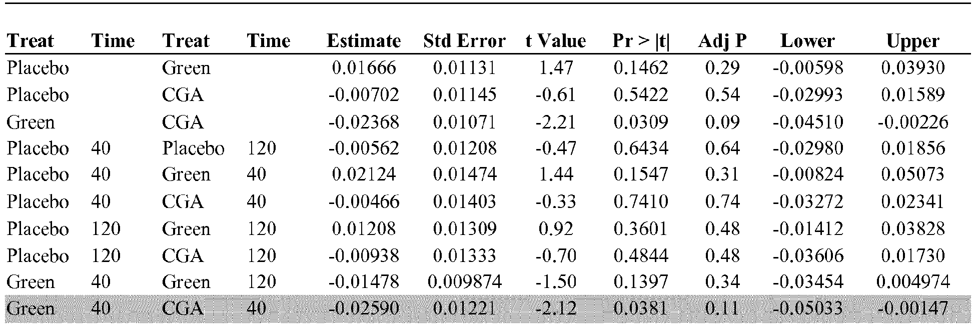

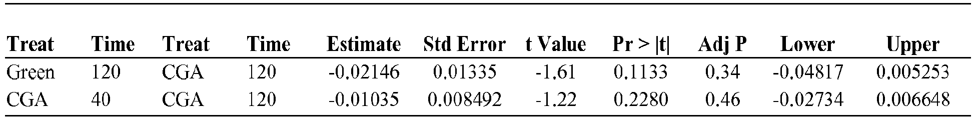

At 40 minutes post-treatment there was a trend towards subjects receiving CGA to display slower Decision Time (DT) in comparison to those receiving Green Blend (p-value=0.11).

### Movement Time

Outlier Removal: Participant Number 8 in the Green Blend condition at 120 minutes was removed due to a 1 millisecond movement time. This movement time is not physiologically feasible, and is due to either anticipatory response or data entry error.

| **Analysis Variable(s) : Jensen Decision Time (ms)** | | | | | | | | | | | | |
|---|---|---|---|---|---|---|---|---|---|---|---|---|
| | | | | | | | | | **Lower 95%** | **Upper 95%** | | |
| | **Treat** | **Time** | **Obs** | **Mean** | **Median** | **N** | **Std Dev** | **Std Error** | **CL for Mean** | **CL for Mean** | **Minimum** | **Maximum** |
| 4-ch | ***Placebo*** | ***Pre*** | 60 | 671.0 | 661.5 | 60 | 114.9 | 14.83 | 641.3 | 700.7 | 386.0 | 968.0 |
| | | ***40*** | 60 | 678.2 | 675.0 | 60 | 109.7 | 14.16 | 649.9 | 706.5 | 402.0 | 956.0 |
| | | ***120*** | 60 | 684.6 | 682.0 | 60 | 116.3 | 15.01 | 654.6 | 714.7 | 420.0 | 1013.0 |
| | ***Green*** | ***Pre*** | 60 | 678.3 | 653.0 | 60 | 113.0 | 14.59 | 649.1 | 707.5 | 476.0 | 915.0 |
| | | ***40*** | 60 | 678.3 | 666.5 | 60 | 111.8 | 14.44 | 649.4 | 707.2 | 475.0 | 944.0 |
| | | ***120*** | 60 | 683.7 | 663.0 | 60 | 112.7 | 14.55 | 654.5 | 712.8 | 464.0 | 946.0 |
| | ***CGA*** | ***Pre*** | 60 | 665.7 | 652.5 | 60 | 109.8 | 14.18 | 637.3 | 694.0 | 422.0 | 991.0 |
| | | ***40*** | 60 | 678.9 | 679.5 | 60 | 108.1 | 13.96 | 651.0 | 706.9 | 388.0 | 909.0 |
| | | ***120*** | 60 | 689.8 | 676.5 | 60 | 114.1 | 14.73 | 660.3 | 719.3 | 468.0 | 972.0 |

Jensen 4-choice. Mixed ANCOVA adjusting for baseline movement time and considering time, treatment and their interaction as independent variables were fitted. The main effect for treatment was approaching significance (F-value=2.58, p-value=0.08) while the Treatment*Time interaction was non-significant (F-value=0.25, p-value=0.78).

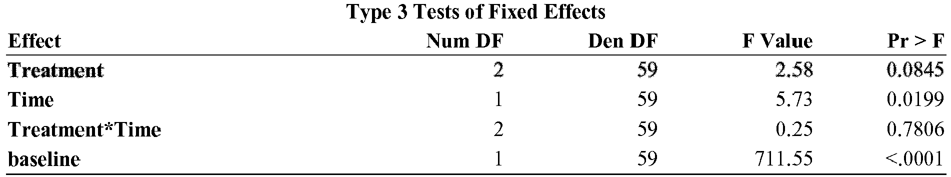

Differences of Least Square Means were calculated between the three treatments as well as the interaction of Treatment*Time. Both unadjusted and adjusted (using Hommel correction) p-values are reported.

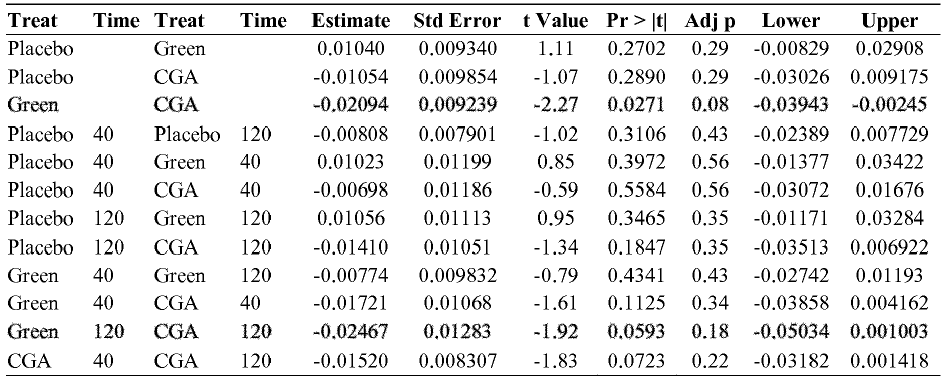

At 120 minutes post-treatment, there was a trend towards subjects consuming Green Blend displaying faster MT in comparison to CGA (p-value=0.18). Overall, there was also a trend towards subjects in the Green Blend group displaying faster MT in comparison to the CGA group (p-value=0.08).

### Bond & Lader Visual Analogue Scales

Outlier Removal: Due to the fact that possible scores are limited between 0 and 100 on all Bond-Lader scales, no outliers were removed from the dataset. However, a number of transformations were required in order to normalize the data.

### Alertness

| **Analysis Variable : Bond-Lader ScoreAlert2** | | | | | | | | | | | |
|---|---|---|---|---|---|---|---|---|---|---|---|
| | | | | | | | | **Lower 95%** | **Upper 95%** | | |
| **Treat** | **Time** | **Obs** | **Mean** | **Median** | **N** | **Std Dev** | **Std Error** | **CL for Mean** | **CL for Mean** | **Minimum** | **Maximum** |
| ***Placebo*** | **Pre** | 60 | 64.06 | 60.50 | 60 | 19.18 | 2.476 | 59.11 | 69.02 | 27.38 | 99.67 |
| | **40min** | 60 | 59.90 | 57.45 | 60 | 21.31 | 2.751 | 54.40 | 65.41 | 13.11 | 99.67 |
| | **120min** | 60 | 56.76 | 55.56 | 60 | 20.48 | 2.644 | 51.47 | 62.05 | 11.89 | 99.89 |
| ***Green Blend*** | **Pre** | 60 | 63.48 | 67.00 | 60 | 19.59 | 2.529 | 58.42 | 68.54 | 19.67 | 100.0 |
| | **40min** | 60 | 61.49 | 60.78 | 60 | 20.12 | 2.598 | 56.29 | 66.69 | 13.33 | 100.0 |
| | **120min** | 60 | 59.51 | 57.00 | 60 | 21.05 | 2.718 | 54.07 | 64.94 | 14.78 | 99.56 |
| ***CGA*** | **Pre** | 60 | 66.29 | 68.11 | 60 | 18.17 | 2.346 | 61.59 | 70.98 | 33.67 | 100.0 |
| | **40min** | 60 | 59.95 | 58.78 | 60 | 20.05 | 2.589 | 54.77 | 65.13 | 14.67 | 99.56 |
| | **120min** | 60 | 57.09 | 60.72 | 60 | 20.24 | 2.613 | 51.86 | 62.31 | 15.33 | 99.67 |

Mixed ANCOVA adjusting for baseline accuracy and considering time, treatment and their interaction as independent variables were fitted. The main effect for treatment was found to be significant (F-value=4.18, p-value=0.02), while the Treatment*Time interaction was found to be non-significant (F-value=0.36, p-value=0.70).

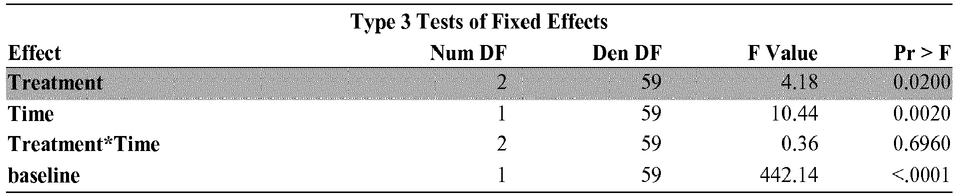

Differences of Least Square Means were calculated between the three treatments as well as the interaction of Treatment*Time. Both unadjusted and adjusted (using Hommel correction) p-values are reported.

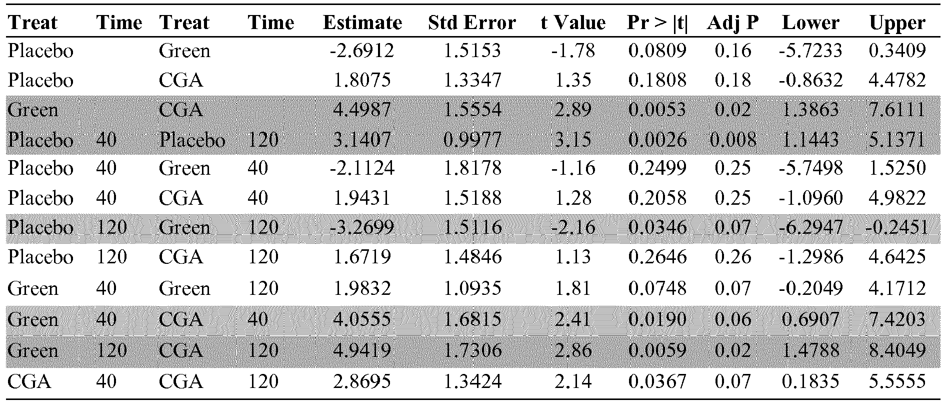

B-L Alertness was found to be significantly higher in the Green Blend group in comparison to CGA at 120 minutes (p-value=0.02). Similarly, there was a trend towards B-L Alertness being higher in the Green Blend group in comparison to placebo at 120 minutes (p-value=0.07). At 40 minutes post-treatment there was a trend towards Alertness being higher in the Green Blend group in comparison to placebo (p-value=0.06). In the placebo group Alertness significantly decreased from 40 to 120 minutes post-treatment (p-value = 0.008). Overall, B-L Alertness was found to be significantly higher after receiving Green Blend in comparison to CGA (p-value=0.02).

### Caffeine Research Visual Analogue Mood Scales (Caff-VAS)

Outlier Removal: As with the Bond-Lader scales, possible scores for the Caff-VAS scales are limited between 0 and 100, and for this reason no outliers were removed from the Caffeine VAS dataset. However, a number of transformations were required in order to normalize the data.

### Alert

| **Analysis Variable : CaffVAS Alert2** | | | | | | | | | | | |
|---|---|---|---|---|---|---|---|---|---|---|---|
| **Treatment** | **Time** | **Obs** | **Mean** | **Median** | **N** | **Std Dev** | **Std Error** | **Lower 95% CL for Mean** | **Upper 95% CL for Mean** | **Minimum** | **Maximum** |
| ***Placebo*** | **Pre** | 60 | 62.67 | 59.50 | 60 | 21.39 | 2.761 | 57.14 | 68.19 | 17.00 | 100.0 |
| | **40min** | 60 | 57.12 | 55.00 | 60 | 21.27 | 2.745 | 51.62 | 62.61 | 9.000 | 100.0 |
| | **120min** | 60 | 51.40 | 48.50 | 60 | 21.99 | 2.839 | 45.72 | 57.08 | 4.000 | 100.0 |
| ***Green Blend*** | **Pre** | 60 | 60.20 | 62.50 | 60 | 22.81 | 2.945 | 54.31 | 66.09 | 19.00 | 100.0 |
| | **40min** | 60 | 58.42 | 57.00 | 59 | 22.66 | 2.950 | 52.52 | 64.33 | 13.00 | 98.00 |
| | **120min** | 60 | 56.65 | 53.50 | 60 | 21.77 | 2.810 | 51.03 | 62.27 | 10.00 | 100.0 |
| ***CGA*** | **Pre** | 60 | 61.95 | 63.50 | 60 | 21.52 | 2.778 | 56.39 | 67.51 | 9.000 | 99.00 |
| | **40min** | 60 | 55.95 | 56.00 | 60 | 24.05 | 3.105 | 49.74 | 62.16 | 3.000 | 100.0 |
| | **120min** | 60 | 49.43 | 46.00 | 60 | 21.65 | 2.795 | 43.84 | 55.03 | 5.000 | 99.00 |

Mixed ANCOVA adjusting for baseline score and considering time, treatment and their interaction as independent variables were fitted. The main effect for treatment was found to be significant (F-value=3.40, p-value=0.04) while the Treatment*Time interaction was found to be non-significant (F-value=2.06, p-value=0.14).

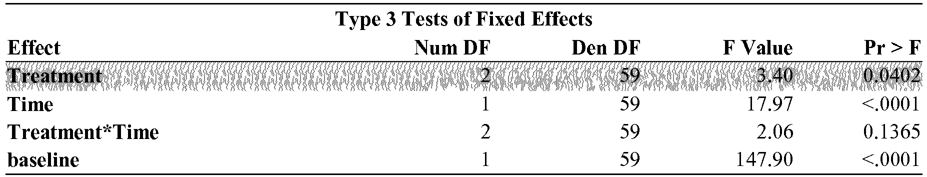

Differences of Least Square Means were calculated between the three treatments as well as the interaction of Treatment*Time. Both unadjusted and adjusted (using Hommel correction) p-values are reported.

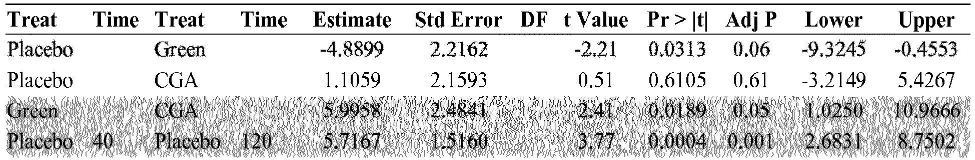

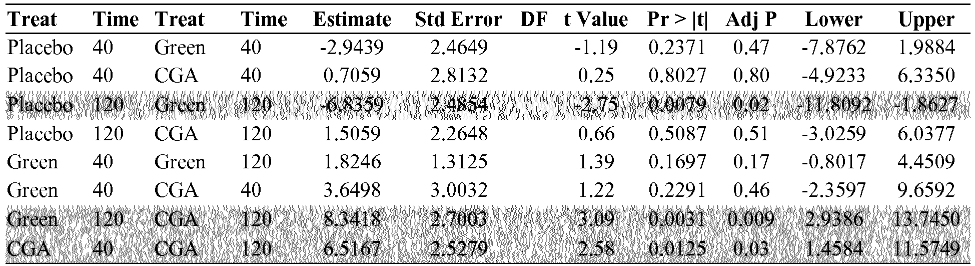

120 minutes after receiving treatments, subjects receiving Green Blend were found to be significantly more Alert than those receiving both placebo (p-value=0.02) and CGA (p-value=0.009). This can be attributed to significant decreases in Alertness from 40 to 120 minutes in both the Placebo group (p-value=0.001) and the CGA group (p-value=0.03). Overall, subjects were found to be significantly more Alert post-treatment after receiving Green Blend in comparison to CGA (p-value=0.05). A similar trend was also observed for Green Blend in comparison to Placebo (p-value=0.06).

### Jittery

### Pre-Testing

| **Analysis Variable : CaffVAS Jittery1** | | | | | | | | | | | |
|---|---|---|---|---|---|---|---|---|---|---|---|
| | | | | | | | | **Lower 95%** | **Upper 95%** | | |
| **Treatment** | **Time** | **Obs** | **Mean** | **Median** | **N** | **Std Dev** | **Std Error** | **CL for Mean** | **CL for Mean** | **Minimum** | **Maximum** |
| ***Placebo*** | ***Pre*** | 60 | 14.45 | 11.50 | 60 | 12.16 | 1.570 | 11.31 | 17.59 | 0 | 55.00 |
| | ***40min*** | 60 | 18.97 | 13.00 | 60 | 17.36 | 2.242 | 14.48 | 23.45 | 0 | 81.00 |
| | ***120min*** | 60 | 17.70 | 13.00 | 60 | 16.59 | 2.142 | 13.41 | 21.99 | 0 | 63.00 |
| ***Green Blend*** | ***Pre*** | 60 | 18.97 | 15.00 | 60 | 16.89 | 2.180 | 14.60 | 23.33 | 0 | 77.00 |
| | ***40min*** | 60 | 16.32 | 12.50 | 60 | 13.85 | 1.789 | 12.74 | 19.90 | 0 | 57.00 |
| | ***120min*** | 60 | 17.10 | 10.00 | 60 | 15.93 | 2.057 | 12.98 | 21.22 | 0 | 65.00 |
| ***CGA*** | ***Pre*** | 60 | 18.73 | 12.50 | 60 | 17.49 | 2.258 | 14.22 | 23.25 | 0 | 86.00 |
| | ***40min*** | 60 | 16.35 | 10.00 | 60 | 15.03 | 1.940 | 12.47 | 20.23 | 0 | 58.00 |
| | ***120min*** | 60 | 18.10 | 14.50 | 60 | 16.38 | 2.115 | 13.87 | 22.33 | 0 | 61.00 |

Mixed ANCOVA adjusting for baseline score and considering time, treatment and their interaction as independent variables were fitted. The main effect for treatment was approaching significance (F-value=2.46, p-value=0.09), while the Treatment*Time interaction was non-significant (F-value=1.43, p-value=0.25).

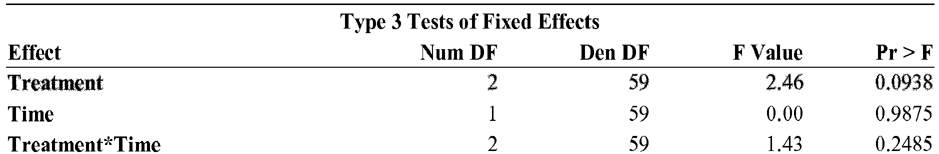

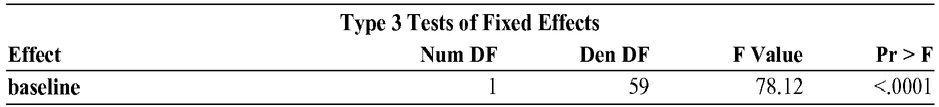

Differences of Least Square Means were calculated between the three treatments as well as the interaction of Treatment*Time. Both unadjusted and adjusted (using Hommel correction) p-values are reported.

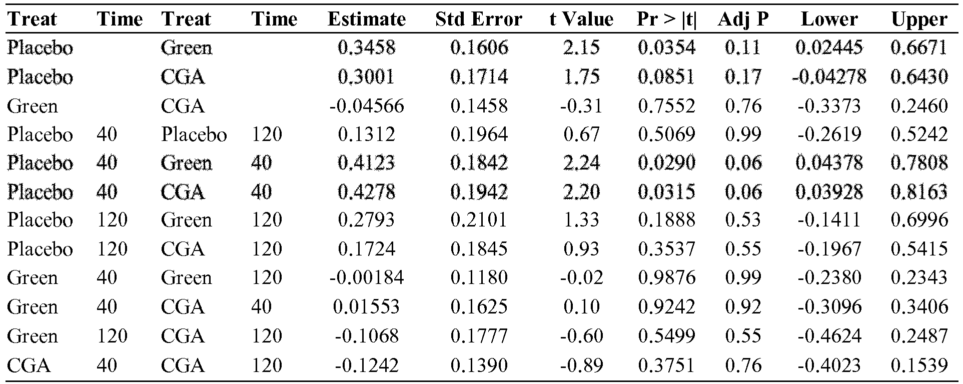

At 40 minutes post-treatment there was a trend towards subjects receiving Green Blend being significantly less jittery than those in the placebo group (p-value=0.06). A similar trend was also observed for the CGA group in comparison to placebo at 40 minutes (p-value=0.06). Overall, CaffVAS-Jittery was found to be lower in the Green Blend treatment in comparison to placebo (p-value=0.11) as well as in the CGA group in comparison to placebo (p-value=0.17).

### Tired

### Pre-Testing

| **Analysis Variable : CaffVAS Tired1** | | | | | | | | | | | |
|---|---|---|---|---|---|---|---|---|---|---|---|
| | | | | | | | | **Lower 95%** | **Upper 95%** | | |
| **Treatment** | **Time** | **Obs** | **Mean** | **Median** | **N** | **Std Dev** | **Std Error** | **CL for Mean** | **CL for Mean** | **Minimum** | **Maximum** |
| ***Placebo*** | **Pre** | 60 | 26.50 | 25.00 | 60 | 17.62 | 2.274 | 21.95 | 31.05 | 0 | 71.00 |
| | **40min** | 60 | 34.03 | 28.50 | 60 | 21.99 | 2.839 | 28.35 | 39.71 | 0 | 81.00 |
| | **120min** | 60 | 40.45 | 39.00 | 60 | 20.27 | 2.616 | 35.21 | 45.69 | 0 | 76.00 |
| ***Green Blend*** | **Pre** | 60 | 26.75 | 24.00 | 60 | 20.10 | 2.595 | 21.56 | 31.94 | 0 | 83.00 |
| | **40min** | 60 | 32.92 | 30.50 | 60 | 20.99 | 2.709 | 27.50 | 38.34 | 0 | 73.00 |
| | **120min** | 60 | 36.25 | 34.00 | 60 | 20.67 | 2.668 | 30.91 | 41.59 | 1.00 | 82.00 |
| ***CGA*** | **Pre** | 60 | 29.62 | 23.00 | 60 | 22.72 | 2.933 | 23.75 | 35.48 | 0 | 84.00 |
| | **40min** | 60 | 33.33 | 29.00 | 60 | 21.18 | 2.735 | 27.86 | 38.81 | 0 | 99.00 |
| | **120min** | 60 | 36.97 | 36.00 | 60 | 20.82 | 2.688 | 31.59 | 42.35 | 1.00 | 82.00 |

Mixed ANCOVA adjusting for baseline score and considering time, treatment and their interaction as independent variables were fitted. The main effect for treatment was found to be non-significant (F-value=.17, p-value=0.32) as well as the Treatment*Time interaction (F-value=0.99, p-value=0.38).

| **Type 3 Tests of Fixed Effects** | | | | |
|---|---|---|---|---|
| **Effect** | **Num DF** | **Den DF** | **F Value** | **Pr > F** |
| **Treatment** | 2 | 59 | 1.17 | 0.3173 |
| **Time** | 1 | 59 | 9.92 | 0.0026 |
| **Treatment*Time** | 2 | 59 | 0.99 | 0.3762 |
| **baseline** | 1 | 59 | 55.76 | <.0001 |

Differences of Least Square Means were calculated between the three treatments as well as the interaction of Treatment*Time. Both unadjusted and adjusted (using Hommel correction) p-values are reported.

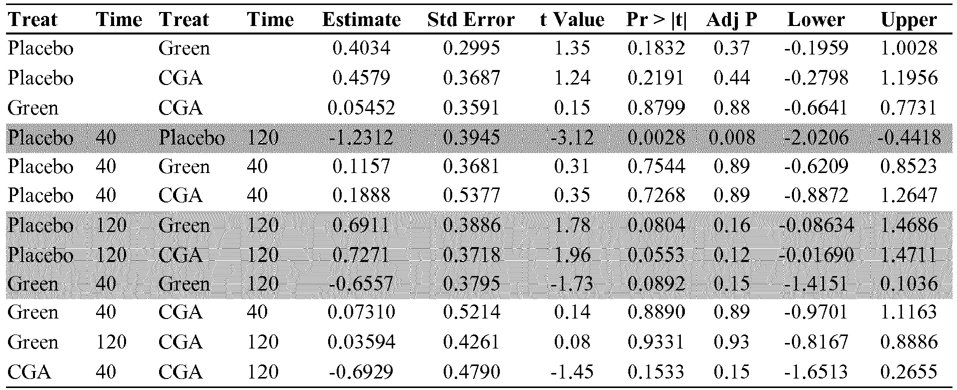

There was a significant increase in Tiredness observed in the Placebo group from 40 to 120 minutes (p-value=0.008). At 120-minutes post-treatment there was a trend towards Tiredness being higher in the placebo in comparison to Green Blend (p-value=0.16) as well as CGA (p-value=0.12).

### Post-testing

| **Analysis Variable : CaffVAS Tired2** | | | | | | | | | | | |
|---|---|---|---|---|---|---|---|---|---|---|---|
| | | | | | | | | **Lower 95%** | **Upper 95%** | | |
| **Treatment** | **Time** | **Obs** | **Mean** | **Median** | **N** | **Std Dev** | **Std Error** | **CL for Mean** | **CL for Mean** | **Minimum** | **Maximum** |
| ***Placebo*** | **Pre** | 60 | 38.38 | 41.50 | 60 | 22.42 | 2.894 | 32.59 | 44.17 | 0 | 94.00 |
| | **40min** | 60 | 47.13 | 49.00 | 60 | 23.40 | 3.021 | 41.09 | 53.18 | 0 | 90.00 |
| | **120min** | 60 | 49.95 | 53.00 | 60 | 21.94 | 2.832 | 44.28 | 55.62 | 1.000 | 91.00 |
| ***Green Blend*** | **Pre** | 60 | 35.98 | 36.50 | 60 | 21.73 | 2.805 | 30.37 | 41.60 | 0 | 81.00 |
| | **40min** | 60 | 44.39 | 45.00 | 59 | 24.81 | 3.230 | 37.92 | 50.86 | 1.000 | 93.00 |
| | **120min** | 60 | 46.63 | 47.00 | 60 | 23.27 | 3.004 | 40.62 | 52.64 | 0 | 92.00 |
| ***CGA*** | **Pre** | 60 | 34.95 | 32.00 | 60 | 21.84 | 2.819 | 29.31 | 40.59 | 0 | 83.00 |
| | **40min** | 60 | 39.80 | 40.50 | 60 | 21.35 | 2.756 | 34.28 | 45.32 | 0 | 87.00 |
| | **120min** | 60 | 50.45 | 51.50 | 60 | 21.76 | 2.809 | 44.83 | 56.07 | 2.000 | 89.00 |

Mixed ANCOVA adjusting for baseline score and considering time, treatment and their interaction as independent variables were fitted. The main effect for treatment was found to be non-significant (F-value=0.50, p-value=0.61) whilst the Treatment*Time interaction was found to be significant (F-value=3.79, p-value=0.03).

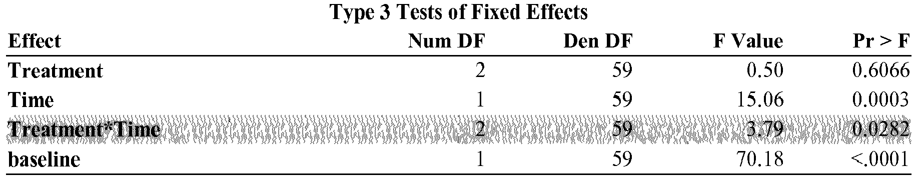

Differences of Least Square Means were calculated between the three treatments as well as the interaction of Treatment*Time. Both unadjusted and adjusted (using Hommel correction) p-values are reported.

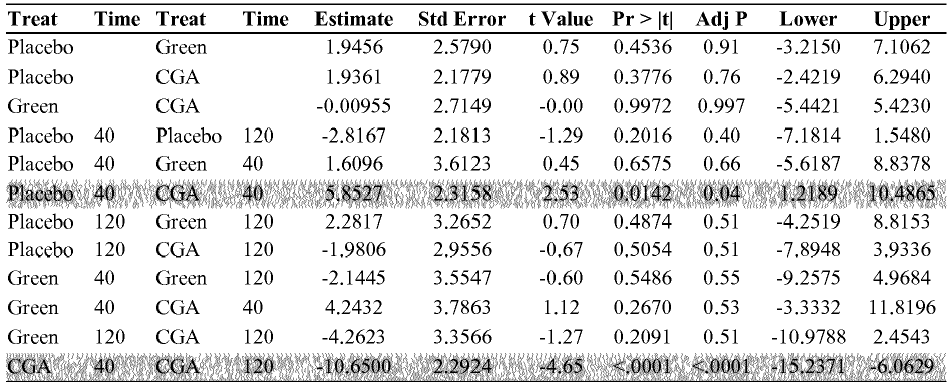

At 40 minutes post-treatment, subjects in the CGA group were found to be significantly less tired than those receiving placebo (p-value=0.04). However, a significant increase in Tiredness was also observed in the CGA group from 40 to 120 minutes post-treatment (p-value<.0001) whilst levels remained relatively unchanged in the placebo group over this time period.

### Tense

### Pre-Testing

| **Analysis Variable : CaffVAS Tense1** | | | | | | | | | | | |
|---|---|---|---|---|---|---|---|---|---|---|---|
| | | | | | | | | **Lower 95%** | **Upper 95%** | | |
| **Treatment** | **Time** | **Obs** | **Mean** | **Median** | **N** | **Std Dev** | **Std Error** | **CL for Mean** | **CL for Mean** | **Minimum** | **Maximum** |
| ***Placebo*** | **Pre** | 60 | 19.85 | 17.00 | 60 | 15.31 | 1.977 | 15.89 | 23.81 | 0 | 75.00 |
| | **40min** | 60 | 20.97 | 16.00 | 60 | 16.40 | 2.117 | 16.73 | 25.20 | 0 | 64.00 |
| | **120min** | 60 | 26.03 | 23.50 | 60 | 18.69 | 2.412 | 21.21 | 30.86 | 1.00 | 69.00 |
| ***Green Blend*** | **Pre** | 60 | 20.78 | 17.00 | 60 | 16.58 | 2.141 | 16.50 | 25.07 | 1.00 | 73.00 |
| | **40min** | 60 | 22.33 | 18.00 | 60 | 19.09 | 2.464 | 17.40 | 27.26 | 0 | 67.00 |
| | **120min** | 60 | 22.47 | 17.00 | 60 | 18.28 | 2.360 | 17.74 | 27.19 | 0 | 62.00 |
| ***CGA*** | **Pre** | 60 | 19.85 | 16.00 | 60 | 15.79 | 2.039 | 15.77 | 23.93 | 1.00 | 69.00 |
| | **40min** | 60 | 21.42 | 19.00 | 60 | 16.26 | 2.099 | 17.22 | 25.62 | 0 | 63.00 |
| | **120min** | 60 | 24.78 | 18.50 | 60 | 19.14 | 2.471 | 19.84 | 29.73 | 0 | 74.00 |

Mixed ANCOVA adjusting for baseline score and considering time, treatment and their interaction as independent variables were fitted. The main effect for treatment was found to be non-significant (F-value=1.06, p-value=0.35), however the Treatment*Time interaction was found to be near-significant (F-value=3.03, p-value=0.06).

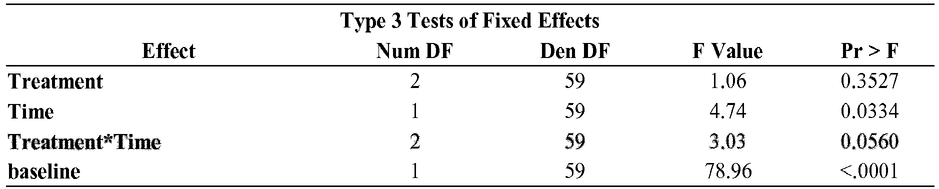

Differences of Least Square Means were calculated between the three treatments as well as the interaction of Treatment*Time. Both unadjusted and adjusted (using Hommel correction) p-values are reported.

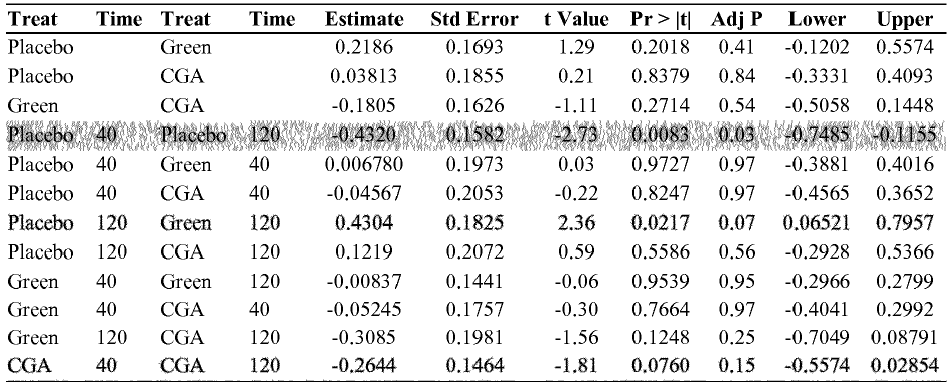

At 120 minutes post-treatment there was a trend towards subjects receiving Green Blend being less tense than those receiving placebo (p-value=0.07). This may be attributable to the significant increase in Caff-VAS Tense score observed from 40 to 120 minutes post-treatment in the placebo group (p-value=0.03) while levels remained relatively stable in the Green Blend group over the same time period. In the CGA group there was also a trend towards an increase in Caff-VAS Tense from 40 to 120 minutes (p-value=0.15).

### Headache

### Post-Testing

| **Analysis Variable : CaffVAS Headache2** | | | | | | | | | | | |
|---|---|---|---|---|---|---|---|---|---|---|---|
| | | | | | | | | **Lower 95%** | **Upper 95%** | | |
| **Treatment** | **Time** | **Obs** | **Mean** | **Median** | **N** | **Std Dev** | **Std Error** | **CL for Mean** | **CL for Mean** | **Minimum** | **Maximum** |
| ***Placebo*** | **Pre** | 60 | 15.03 | 7.000 | 60 | 18.09 | 2.335 | 10.36 | 19.71 | 0 | 65.00 |
| | **40min** | 60 | 16.38 | 9.000 | 60 | 19.00 | 2.453 | 11.47 | 21.29 | 0 | 78.00 |
| | **120min** | 60 | 21.22 | 9.500 | 60 | 23.05 | 2.976 | 15.26 | 27.17 | 0 | 80.00 |
| ***Green Blend*** | **Pre** | 60 | 18.98 | 8.000 | 60 | 23.90 | 3.086 | 12.81 | 25.16 | 0 | 89.00 |
| | **40min** | 60 | 17.68 | 8.000 | 59 | 22.92 | 2.983 | 11.71 | 23.65 | 0 | 81.00 |
| | **120min** | 60 | 19.95 | 5.000 | 60 | 25.91 | 3.345 | 13.26 | 26.64 | 0 | 87.00 |
| ***CGA*** | **Pre** | 60 | 17.18 | 10.00 | 60 | 17.94 | 2.317 | 12.55 | 21.82 | 0 | 64.00 |
| | **40min** | 60 | 18.20 | 9.500 | 60 | 20.24 | 2.613 | 12.97 | 23.43 | 0 | 75.00 |
| | **120min** | 60 | 17.18 | 8.000 | 60 | 19.28 | 2.489 | 12.20 | 22.16 | 0 | 69.00 |

Mixed ANCOVA adjusting for baseline score and considering time, treatment and their interaction as independent variables were fitted. The main effect for treatment was found to be non-significant (F-value=1.92, p-value=0.16) whilst the Treatment*Time interaction was found to be significant (F-value=5.93, p-value<.01).

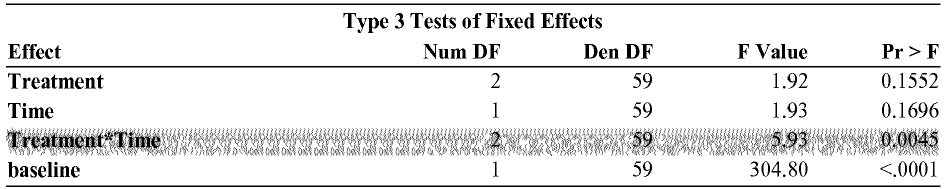

Differences of Least Square Means were calculated between the three treatments as well as the interaction of Treatment*Time. Both unadjusted and adjusted (using Hommel correction) p-values are reported.

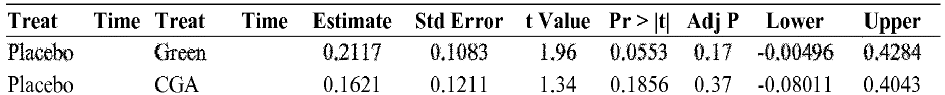

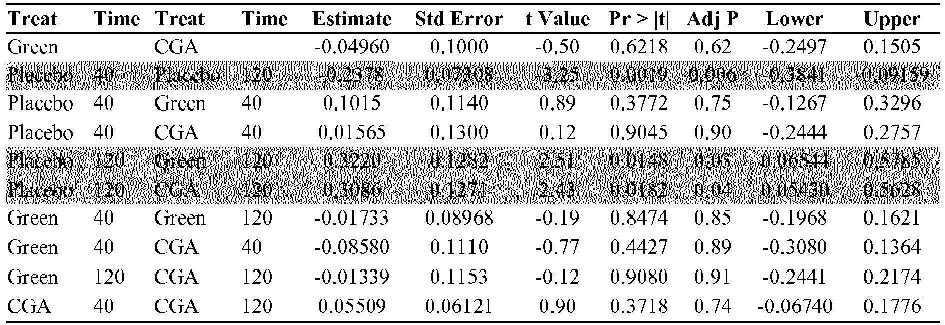

From 40 to 120 minutes post-treatment there was a significant increase in headache symptoms in the placebo group (p-value=0.006). At 120 minutes post-treatment subjects receiving Green Blend were found to report significantly less headache symptoms in comparison to placebo (p-value=0.03). Similarly, subjects receiving CGA were also found to report significantly less headache symptoms in comparison to placebo at 120 minutes (p-value=0.04). Overall there was a trend towards subjects receiving Green blend reporting significantly less headache symptoms in comparison to placebo (p-value=0.17).

### Mental Fatigue

### Pre-Testing

| **Analysis Variable : CaffVAS MentFatiguel** | | | | | | | | | | | |
|---|---|---|---|---|---|---|---|---|---|---|---|
| | | | | | | | | **Lower 95%** | **Upper 95%** | | |
| **Treatment** | **Time** | **Obs** | **Mean** | **Median** | **N** | **Std Dev** | **Std Error** | **CL for Mean** | **CL for Mean** | **Minimum** | **Maximum** |
| ***Placebo*** | **Pre** | 60 | 21.65 | 18.00 | 60 | 17.08 | 2.205 | 17.24 | 26.06 | 0 | 77.00 |
| | **40min** | 60 | 31.72 | 28.00 | 60 | 21.59 | 2.787 | 26.14 | 37.29 | 1.000 | 77.00 |
| | **120min** | 60 | 37.08 | 36.00 | 60 | 20.84 | 2.690 | 31.70 | 42.47 | 1.000 | 80.00 |
| ***Green Blend*** | **Pre** | 60 | 21.73 | 15.00 | 60 | 19.21 | 2.480 | 16.77 | 26.70 | 0 | 73.00 |
| | **40min** | 60 | 32.62 | 26.50 | 60 | 23.45 | 3.028 | 26.56 | 38.68 | 0 | 92.00 |
| | **120min** | 60 | 37.42 | 30.00 | 60 | 24.63 | 3.179 | 31.05 | 43.78 | 0 | 97.00 |
| ***CGA*** | **Pre** | 60 | 22.75 | 14.00 | 60 | 20.65 | 2.666 | 17.42 | 28.08 | 0 | 87.00 |
| | **40min** | 60 | 29.73 | 23.50 | 60 | 21.39 | 2.761 | 24.21 | 35.26 | 0 | 73.00 |
| | **120min** | 60 | 37.77 | 31.00 | 60 | 25.79 | 3.329 | 31.10 | 44.43 | 2.000 | 96.00 |

Mixed ANCOVA adjusting for baseline score and considering time, treatment and their interaction as independent variables were fitted. The main effect for treatment was found to be non-significant (F-value=0.38, p-value=0.69) as well as the Treatment*Time interaction (F-value=0.28, p-value=0.76).

| **Type 3 Tests of Fixed Effects** | | | | |
|---|---|---|---|---|
| **Effect** | **Num DF** | **Den DF** | **F Value** | **Pr > F** |
| **Treatment** | 2 | 59 | 0.38 | 0.6856 |
| **Time** | 1 | 59 | 14.64 | 0.0003 |
| **Treatment*Time** | 2 | 59 | 0.28 | 0.7577 |
| **baseline** | 1 | 59 | 70.32 | <.0001 |

Differences of Least Square Means were calculated between the three treatments as well as the interaction of Treatment*Time. Both unadjusted and adjusted (using Hommel correction) p-values are reported.

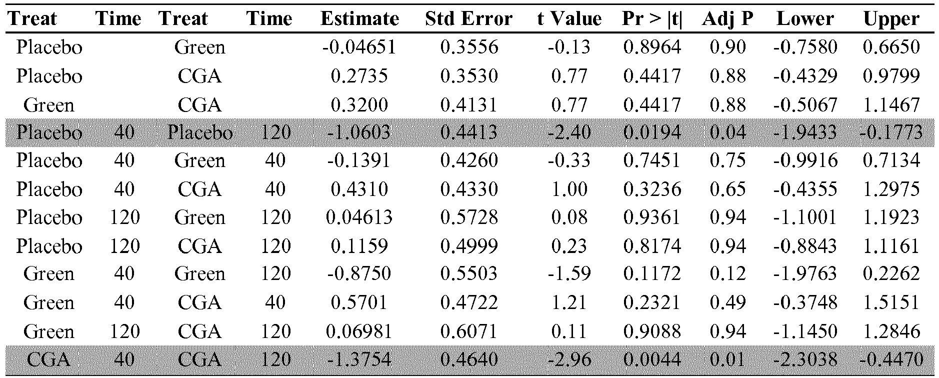

No significant treatment effects were observed, although significant increases in mental fatigue were observed from 40 to 120 minutes in both the Placebo (p-value=0.04) and CGA group (p-value=0.01).

### Post-Testing

| **Analysis Variable : CaffVAS MentFatigue2** | | | | | | | | | | | |
|---|---|---|---|---|---|---|---|---|---|---|---|
| **Treatment** | **Time** | **Obs** | **Mean** | **Median** | **N** | **Std Dev** | **Std Error** | **Lower 95% CL for Mean** | **Upper 95% CL for Mean** | **Minimum** | **Maximum** |
| ***Placebo*** | **Pre** | 60 | 37.58 | 35.00 | 60 | 24.06 | 3.106 | 31.37 | 43.80 | 0 | 94.00 |
| | **40min** | 60 | 44.77 | 42.00 | 60 | 24.12 | 3.114 | 38.53 | 51.00 | 0 | 90.00 |
| | **120min** | 60 | 52.02 | 59.00 | 60 | 25.01 | 3.228 | 45.56 | 58.48 | 0 | 94.00 |
| ***Green Blend*** | **Pre** | 60 | 38.22 | 36.50 | 60 | 24.32 | 3.139 | 31.94 | 44.50 | 0 | 87.00 |
| | **40min** | 60 | 45.31 | 45.00 | 59 | 25.36 | 3.302 | 38.70 | 51.91 | 1.000 | 92.00 |
| | **120min** | 60 | 48.67 | 56.00 | 60 | 25.51 | 3.294 | 42.08 | 55.26 | 2.000 | 94.00 |
| ***CGA*** | **Pre** | 60 | 37.22 | 34.50 | 60 | 23.72 | 3.062 | 31.09 | 43.34 | 0 | 80.00 |
| | **40min** | 60 | 45.62 | 46.50 | 60 | 24.02 | 3.101 | 39.41 | 51.82 | 1.000 | 91.00 |
| | **120min** | 60 | 53.55 | 57.50 | 60 | 24.00 | 3.098 | 47.35 | 59.75 | 0 | 94.00 |

Mixed ANCOVA adjusting for baseline score and considering time, treatment and their interaction as independent variables were fitted. The main effect for treatment was found to be non-significant (F-value=0.88, p-value=0.42) as well as the Treatment*Time interaction (F-value=0.72, p-value=0.49).

| **Type 3 Tests of Fixed Effects** | | | | |
|---|---|---|---|---|
| **Effect** | **Num DF** | **Den DF** | **F Value** | **Pr > F** |
| **Treatment** | 2 | 59 | 0.88 | 0.4183 |
| **Time** | 1 | 59 | 15.82 | 0.0002 |
| **Treatment*Time** | 2 | 59 | 0.72 | 0.4886 |
| **baseline** | 1 | 59 | 72.23 | <.0001 |

Differences of Least Square Means were calculated between the three treatments as well as the interaction of Treatment*Time. Both unadjusted and adjusted (using Hommel correction) p-values are reported.

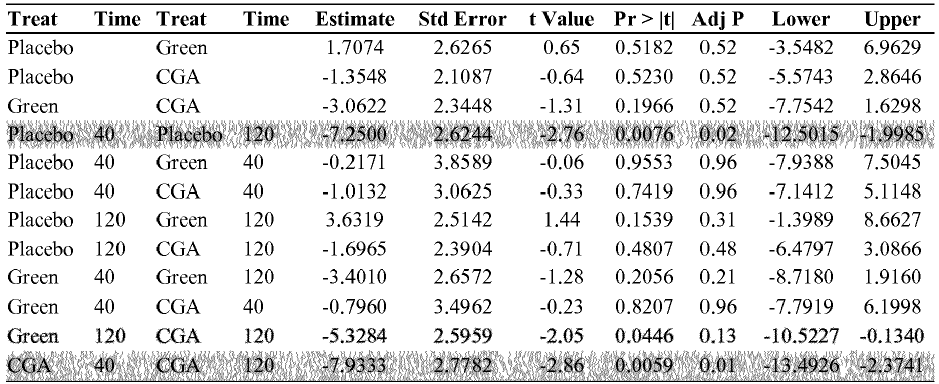

There was a significant increase in ratings of mental fatigue from 40 to 120 minutes in both placebo (p-value=0.02) and CGA groups (p-value=0.01), whilst levels remained relatively unchanged in the Green Blend group over this time period. At 120 minutes post-treatment there was a trend towards subj ects receiving Green Blend were reporting less mental fatigued in comparison to those receiving CGA (p-value=0.13).
The invention is claimed as follows:

## Claims

1. A method of improving alertness, improving attention, reducing tension, and/or reducing mental fatigue in an individual for at least 1 hour, the method comprising:
administering to an individual in need of improved alertness, improved attention, reduced tension, and/or reduced mental fatigue a decaffeinated coffee product to improve alertness, improve attention, reduce tension, and/or reduce mental fatigue in the individual.

2. The method of Claim 1, wherein the decaffeinated coffee product has a similar effect in the individual as an approximately equivalent amount of a caffeinated coffee product.

3. The method of any one of Claims 1 to 2, wherein the decaffeinated coffee product comprises a mixture of unroasted coffee and roasted coffee.

4. The method of any one of Claims 1 to 3, wherein the decaffeinated coffee product comprises an extract of unroasted coffee.

5. The method of Claim 4, wherein the extract is derived from:
(i) a first portion comprising unroasted ground and/or unground coffee, in an amount of from 1 to 90% by weight based on the total weight of the coffee product, and
(ii) a second portion comprising ground coffee that has been roasted to a higher degree of roast than the first portion, in an amount of from 99 to 10% by weight based on the total weight of the coffee product, wherein the content of the chlorogenic acids is at least 4 g per 100 g of the coffee product.

6. A method of improving reaction time, reducing tiredness and/or reducing jitteriness in an individual, the method comprising:
administering to an individual in need of improved reaction time, reduced tiredness and/or reduced jitteriness a decaffeinated coffee product to improve reaction time, reduce tiredness and/or reduce jitteriness in the individual.

7. The method of Claim 6, wherein the decaffeinated coffee product has a similar effect in the individual as an approximately equivalent amount of a caffeinated coffee product.

8. The method of any one of Claims 6 to 7, wherein the decaffeinated coffee product comprises a mixture of unroasted coffee and roasted coffee.

9. The method of any one of Claims 6 to 8, wherein the decaffeinated coffee product comprises an extract of unroasted coffee.

10. The method of Claim 9, wherein the extract is derived from:
(i) a first portion comprising unroasted ground and/or unground coffee, in an amount of from 1 to 90% by weight based on the total weight of the coffee product, and
(ii) a second portion comprising ground coffee that has been roasted to a higher degree of roast than the first portion, in an amount of from 99 to 10% by weight based on the total weight of the coffee product, wherein the content of the chlorogenic acids is at least 4 g per 100 g of the coffee product.

11. A method of reducing headache for at least 1 hour in an individual, the method comprising:
administering to an individual in need of reduced headache a decaffeinated coffee product to reduce headache in the individual.

12. The method of Claim 11, wherein the decaffeinated coffee product has a similar effect in the individual as an approximately equivalent amount of a caffeinated coffee product.

13. The method of any one of Claims 11 to 12, wherein the decaffeinated coffee product comprises a mixture of unroasted coffee and roasted coffee.

14. The method of any one of Claims 11 to 13, wherein the decaffeinated coffee product comprises an extract of an unroasted coffee.

15. The method of Claim 14, wherein the extract is derived from:
(i) a first portion comprising unroasted ground and/or unground coffee, in an amount of from 1 to 90% by weight based on the total weight of the coffee product, and
(ii) a second portion comprising ground coffee that has been roasted to a higher degree of roast than the first portion, in an amount of from 99 to 10% by weight based on the total weight of the coffee product, wherein the content of the chlorogenic acids is at least 4 g per 100 g of the coffee product.

16. A method of improving alertness in an individual for at least 1 hour, the method comprising:
administering to an individual in need of improved alertness a non-coffee product comprising a component selected from the group consisting of a decaffeinated coffee extract, chlorogenic acids and combinations thereof to improve alertness in the individual.

17. The method of Claim 21, wherein the chlorogenic acids are derived from a food source.

18. A method of reducing jitteriness in an individual, the method comprising:
administering to an individual in need of reduced jitteriness a non-coffee product comprising a component selected from the group consisting of a decaffeinated coffee extract, chlorogenic acids and combinations thereof to reduce jitteriness of the individual.

19. The method of Claim 23, wherein the chlorogenic acids are derived from a food source.

20. A method of reducing headache for at least 1 hour in an individual, the method comprising:
administering to an individual in need of reduced headache a non-coffee product comprising a component selected from the group consisting of a decaffeinated coffee extract, chlorogenic acids and combinations thereof to reduce headache of the individual.

21. The method of Claim 25, wherein the chlorogenic acids are derived from a food source.

22. The use of a decaffeinated coffee product to improve alertness, improve attention, reduce tension, and/or reduce mental fatigue in an individual for at least 1 hour.

23. The use of claim 22 wherein the decaffeinated coffee product comprises an extract of unroasted coffee.

24. The use of a decaffeinated coffee product to reduce tiredness, improve reaction time and/or reduce jitteriness in an individual.

25. The use of claim 24 wherein the decaffeinated coffee product comprises an extract of unroasted coffee.

26. The use of chlorogenic acids to reduce jitteriness in an individual.

27. The use of chlorogenic acids to reduce headache in an individual for at least 1 hour.

28. The use of chlorogenic acids to reducetiredness in an individual for at least 1 hour.
